# EUROPÄISCHE PATENTANMELDUNG

(11) **EP 4 491 140 A1**
(43) Veröffentlichungstag der Anmeldung: **15.01.2025**
(21) Anmeldenummer: 24182068.7
(22) Anmeldetag: 13.06.2024
(51) Int. Cl.: A61B 17/88, A61B 90/00

(54) **DREHMOMENTBEGRENZER SOWIE GRIFFEINHEIT UND CHIRURGISCHES INSTRUMENT MIT EINEM DREHMOMENTBEGRENZER**

(30) Priorität: 13.07.2023 DE 102023118540
(71) Anmelder: Wernz, Ulrich, 78532 Tuttlingen (DE)
(72) Erfinder: Wernz, Ulrich, 78532 Tuttlingen (DE)
(74) Vertreter: Westphal, Mussgnug & Partner Patentanwälte mbB

(57) **Zusammenfassung**

Drehmomentbegrenzer (18), insbesondere für ein chirurgisches Instrument, mit einer Rotationsachse (30), einem Einleitelement (20) zum Einleiten von Drehmoment in den Drehmomentbegrenzer (18), einem Ausleitelement (22) zum Ausleiten von Drehmoment aus dem Drehmomentbegrenzer (18), und einer mindestens einen Kupplungskörper (32) aufweisenden Kupplungseinheit (24), mittels der das Einleitelement (20) mit dem Ausleitelement (22) koppelbar ist, wobei die Kupplungseinheit (24) auswechselbar in dem Drehmomentbegrenzer (18) angeordnet ist, sowie
Griffeinheit (10) mit einem solchen Drehmomentbegrenzer (18) und chirurgisches Instrument mit einem solchen Drehmomentbegrenzer (18) oder einer solchen Griffeinheit (10).

## Beschreibung

Die Erfindung betrifft einen Drehmomentbegrenzer sowie eine Griffeinheit und ein chirurgisches Instrument mit einem solchen Drehmomentbegrenzer.

Drehmomentbegrenzer sind in vielerlei Ausführungsformen bekannt. Derartige Drehmomentbegrenzer weisen eine Drehmomentgrenze auf, die festgelegt oder einstellbar sein kann. Üblicherweise ist ein Drehmomentbegrenzer dabei derart konfiguriert, dass ein eingangsseitig auf den Drehmomentbegrenzer aufgebrachtes Drehmoment nur bis zur Drehmomentgrenze auf eine Ausgangsseite des Drehmomentbegrenzers übertragen wird.

Gerade bei medizinischen Anwendungen ist es häufig wichtig, genaue Drehmomentvorgaben einzuhalten, um die Sicherheit von Implantaten und anderen Medizinprodukten zu gewährleisten. Drehmomentbegrenzer kommen daher auch in chirurgischen Instrumenten zum Einsatz. Derartige Drehmomentbegrenzer sind beispielsweise aus der EP 2 311 397 B1 und der EP 2 085 041 A1 bekannt.

Nachteilig an den bekannten Drehmomentbegrenzern ist, dass die Drehmomentgenauigkeit mit der Zeit derart nachlässt, dass die Ist-Drehmomentgrenze in nicht mehr akzeptabler Weise von der Soll-Drehmomentgrenze abweicht. Gerade bei chirurgischen Drehmomentbegrenzern ist jedoch eine hohe Drehmomentgenauigkeit erforderlich.

Für chirurgische Instrumente mit Drehmomentbegrenzern wird daher eine maximale Lebensdauer, beispielsweise in Abhängigkeit ihres Alters oder der erlebten Sterilisationszyklen, festgelegt, nach deren Erreichen die Instrumente nicht mehr verwendet werden dürfen und daher rekalibriert oder entsorgt werden müssen.

Dies führt nicht nur zu einer großen Menge anfallenden Instrumentenschrotts sondern auch zu hohen Kosten, insbesondere da es sich bei Drehmomentbegrenzer für chirurgische Instrumente meist um sehr hochwertige Systeme handelt.

Der Erfindung liegt daher die Aufgabe zugrunde, einen Drehmomentbegrenzer bereitzustellen, mit dem die Standzeit von chirurgischen Instrumenten erhöht werden kann, und der einfach und kostengünstig herstellbar ist.

Der Erfindung liegt ferner die Aufgabe zugrunde, eine Griffeinheit sowie ein chirurgisches Instrument mit den entsprechenden Eigenschaften bereitzustellen.

Die Aufgabe wird erfindungsgemäß gelöst durch einen Drehmomentbegrenzer mit den Merkmalen des Patentanspruchs 1, eine Griffeinheit mit den Merkmalen des Patentanspruchs 13 sowie ein chirurgisches Instrument mit den Merkmalen des Patentanspruchs 15.

Vorteilhafte Ausgestaltungen und Weiterbildungen der Erfindung sind in den abhängigen Ansprüchen angegeben.

Ein erfindungsgemäßer Drehmomentbegrenzer, insbesondere für ein chirurgisches Instrument, umfasst eine Rotationsachse, ein Einleitelement zum Einleiten von Drehmoment in den Drehmomentbegrenzer, ein Ausleitelement zum Ausleiten von Drehmoment aus dem Drehmomentbegrenzer, und eine mindestens einen Kupplungskörper aufweisenden Kupplungseinheit, mittels der das Einleitelement mit dem Ausleitelement koppelbar ist, wobei die Kupplungseinheit auswechselbar in dem Drehmomentbegrenzer angeordnet ist. Dadurch kann nach Erreichen einer festgelegten Lebensdauer, beispielsweise einem bestimmten Alter oder einer bestimmten Anzahl an Sterilisationszyklen, die Kupplungseinheit gegen eine neue, kalibrierte Kupplungseinheit ausgetauscht werden. Dadurch kann die für die Festlegung einer tatsächlichen Drehmomentgrenze wesentliche Baugruppe erneuert werden, ohne dass der gesamte Drehmomentbegrenzer beziehungsweise das gesamte chirurgische Instrument entsorgt werden müsste.

Vorzugsweise ist der Drehmomentbegrenzer derart ausgebildet, dass ein auf das Einleitelement aufgebrachtes Drehmoment unterhalb der Drehmomentgrenze vom Einleitelement auf das Ausleitelement übertragbar ist. Ein die Drehmomentgrenze übersteigender Betrag des am Einleitelement anliegenden Drehmoments ist vorzugsweise hingegen nicht von dem Einleitelement auf das Ausleitelement übertragbar. Der Kupplungskörper kann zur kraft- und/oder formschlüssigen Drehmomentübertragung ausgebildet sein.

Bevorzugt ist die Kupplungseinheit ohne spezielle Montagevorrichtung und/oder mit Standardwerkzeug auswechselbar. Die Kupplungseinheit kann insbesondere werkzeuglos auswechselbar sein. Besonders bevorzugt ist die Kupplungseinheit anwenderseitig auswechselbar in dem Drehmomentbegrenzer angeordnet. Sofern der Drehmomentbegrenzer für ein chirurgisches Instrument geeignet und/oder vorgesehen ist, kann dabei medizinisches Fachpersonal als durchschnittlicher Anwenderkreis angenommen werden. Vorzugsweise ist die Kupplungseinheit vollständig, also mit allen zu der Kupplungseinheit zählenden Komponenten, auswechselbar ausgebildet.

Die Kupplungseinheit kann eine, um die Rotationsachse angeordnete, kreisringförmige Grundform aufweisen. Vorzugsweise weist damit auch der mindestens eine Kupplungskörper eine kreisringförmige Grundform auf. Bevorzugt ist das Einleitelement bezüglich der Rotationsachse radial außerhalb der Kupplungseinheit, das Ausleitelement bezüglich der Rotationsachse radial innerhalb der Kupplungseinheit angeordnet.

Die Kupplungseinheit kann entlang der Rotationsachse in und/oder an dem Drehmomentbegrenzer angeordnet sein. Dadurch kann der konstruktive Aufbau des Drehmomentbegrenzers vereinfacht werden.

Vorzugsweise weist die Kupplungseinheit ein erstes Kupplungselement auf, das einerseits mit dem mindestens einen Kupplungskörper sowie andererseits mit dem Einleitelement oder dem Ausleitelement drehfest und/oder unmittelbar koppelbar ist. Dabei ist das erste Kupplungselement vorzugsweise durch ein von dem Einleitelement verschiedenen Bauteil gebildet. Insbesondere wenn das erste Kupplungselement radial außerhalb des mindestens einen Kupplungskörpers angeordnet ist, kann das erste Kupplungselement für die Kupplungseinheit eine Gehäusefunktion aufweisen. Dadurch kann die Auswechselbarkeit der Kupplungseinheit verbessert werden. Die drehfeste Kopplung kann jeweils in Form einer kraft- und/oder formschlüssigen Verbindung realisiert sein. Eine bevorzugte Ausführungsform einer drehfesten Kopplung ist dabei eine formschlüssige Verbindung, insbesondere mittels einer Keilverzahnung.

Die Kupplungseinheit kann ferner ein zweites Kupplungselement aufweisen, das einerseits mit dem mindestens einen Kupplungskörper drehmomentabhängig und/oder unmittelbar sowie andererseits mit dem Einleitelement oder dem Ausleitelement drehfest und/oder unmittelbar koppelbar ist. Durch die drehmomentabhängige Kopplung kann mittels der Wirkverbindung des Kupplungskörpers mit dem zweiten Kupplungselement die Drehmomentgrenze des Drehmomentbegrenzers definiert sein. Die drehmomentabhängige Kopplung kann dabei mittels einer, vorzugsweise unmittelbaren, form- und/oder kraftschlüssigen Verbindung realisiert sein, die vorzugsweise bei Überschreiten der Drehmomentgrenze nach dem Prinzip einer Rutschkupplung aufgehoben wird. Durch das Vorsehen des zweiten Kupplungselements kann insbesondere der dabei auftretende Verschleiß an dem zweiten Kupplungselement stattfinden, sodass die Lebensdauer des Einleit- bzw. des Ausleitelements dadurch nicht beeinträchtigt wird. Die drehfeste Kopplung des mindestens einen Kupplungskörpers mit dem Einleitelement oder dem Ausleitelement ist vorzugsweise durch eine formschlüssige Verbindung, beispielsweise mittels einer Keilverzahnung, realisiert.

In einer bevorzugten Ausführungsform der Erfindung weist der mindestens eine Kupplungskörper mindestens ein, vorzugsweise als Federarm ausgebildetes, Andrückelement auf. Bevorzugt liegt das mindestens eine Andrückelement an dem zweiten Kupplungselement an und stellt so die Wirkverbindung zwischen dem mindestens einen Kupplungskörper und dem zweiten Kupplungselement her. Der Federarm kann sich durch einen Spalt, der insbesondere L-förmig ausgebildet sein kann, von dem übrigen Kupplungskörper abheben. Mittels des Spalts ist vorzugsweise die federnde Charakteristik des Federarms realisiert. Der Kupplungskörper kann eine Vielzahl von Andrückelementen aufweisen.

Das zweite Kupplungselement kann mindestens ein, vorzugsweise als Vertiefung ausgebildetes, Rastelement zum Zusammenwirken mit dem mindestens einen Andrückelement aufweisen. Die Kontur des mindestens einen Andrückelements entspricht vorzugsweise zumindest abschnittsweise der Kontur des mindestens einen Rastelements. Das zweite Kupplungselement kann eine Vielzahl an Rastelementen aufweisen. Bevorzugt weist das zweite Kupplungselement mindestens genauso viele Rastelemente auf wie der mindestens eine Kupplungskörper Andrückelemente. Die Anzahl der Rastelemente des zweiten Kupplungselements kann alternativ ein ganzzahlig Vielfaches der Anzahl an Andrückelementen des mindestens einen Kupplungskörpers sein.

Gemäß einer alternativen Ausführungsform der Erfindung ist das mindestens eine Rastelement als Walzenkörper ausgebildet, der in mindestens einer Ausnehmung des zweiten Kupplungselements angeordnet ist. Dabei ist vorzugsweise in jeder der mindestens einen Ausnehmung einer des mindestens einen Walzenkörpers angeordnet. Der mindestens eine Walzenkörper kann drehbar in der mindestens einen Ausnehmung angeordnet sein. Der mindestens eine Walzenkörper ist vorzugsweise zylindrisch ausgebildet. Der mindestens eine Walzenkörper und/oder die mindestens eine Ausnehmung ist vorzugsweise parallel zu der Rotationsachse des Drehmomentbegrenzers angeordnet. Das zweite Kupplungselement kann mehrere dem mindestens einen Walzenkörper entsprechende Walzenkörper aufweisen, die vorzugsweise gleichmäßig verteilt um die Rotationsachse angeordnet sind.

Das mindestens eine Andrückelement kann mit dem mindestens einen Rastelement in unmittelbare Wirkverbindung bringbar sein. Dadurch können die Teileanzahl und das Gewicht des Drehmomentbegrenzers reduziert werden. Durch die geringere Teileanzahl kann insbesondere die Auswechselbarkeit der Kupplungseinheit vereinfacht oder überhaupt erst ermöglicht werden, beispielsweise indem lose Wälzkörper vermieden werden. Eine permanente Wirkverbindung zwischen dem mindestens einen Andrückelement und dem mindestens einen Rastelement besteht vorzugsweise in einem eingerasteten Zustand, also unterhalb der Drehmomentgrenze.

In einer bevorzugten Ausführungsform der Erfindung ist das mindestens eine Andrückelement durch eine Vielzahl von Andrückelementen gebildet, die sämtlich in einer selben Umfangsrichtung um die Rotationsachse ausgerichtet sind. Insbesondere, wenn das mindestens eine Andrückelement als Federarm ausgebildet ist, sind damit alle Federärme in derselben Umfangrichtung ausgerichtet. So kann eine erste Drehmomentgrenze in einer ersten Umfangsrichtung um die Rotationsachse gegenüber einer zweiten Drehmomentgrenze in einer zweiten Umfangsrichtung um die Rotationsachse unterschiedlich hoch ausgebildet sein. Wird der Drehmomentbegrenzer beispielsweise in einem Schraubendreher eingesetzt, kann so zum Beispiel ein maximales Anzugsmoment kleiner festgelegt werden als ein maximales Lösemoment.

Der mindestens eine Kupplungskörper kann durch mehrere Kupplungskörper gebildet sein, die entlang der Rotationsachse nacheinander angeordnet sind. Dadurch kann die Herstellung und Montage der Kupplungseinheit vereinfacht werden. Durch die Anzahl der verwendeten Kupplungskörper kann, insbesondere werkseitig, die Drehmomentgrenze einstellbar sein. An mindestens einem axialen Ende der Kupplungseinheit kann ein Sicherungsring zur axialen Sicherung des mindestens einen Kupplungskörpers angeordnet sein.

In einer Weiterbildung der Erfindung umfasst die Kupplungseinheit mindestens ein, vorzugsweise als Gummiring ausgebildetes, Distanzelement, das entlang der Rotationsachse angrenzend an einen des mindestens einen Kupplungskörpers angeordnet ist. Das mindestens eine Distanzelement kann zumindest abschnittsweise im Wechsel mit dem mindestens einen Kupplungskörper angeordnet sein. Das mindestens eine Distanzelement kann zur Dämpfung, insbesondere des mindestens einen Andrückelements, sowie zur Geräuschminderung beitragen. Außerdem kann es eine, zumindest leichte, Erhöhung der Drehmomentgrenze bewirken. Darüber hinaus kann das Distanzelement eine axiale Montagesicherung für den mindestens einen Drehmomentkörper darstellen und damit der Montageerleichterung dienen.

Die Kupplungseinheit kann derart ausgebildet sein, dass sie eine im Wesentlichen pilzförmige Außenkontur aufweist. Ein Hutteil der pilzförmigen Außenkontur kann besonders gut zu greifen sein, was die Auswechselbarkeit der Kupplungseinheit erleichtern kann. Insbesondere, wenn die Kupplungseinheit werkzeuglos auswechselbar ausgebildet ist, weist sie vorzugsweise eine pilzförmige Kontur auf. Die pilzförmige Außenkontur ist vorzugsweise dadurch gebildet, dass eines der Kupplungselemente, besonders bevorzugt das erste Kupplungselement, pilzförmig ausgebildet ist.

In einer Ausführungsform der Erfindung ist der Hutteil axial abnehmbar an dem ersten Kupplungselement oder dem zweiten Kupplungselement angeordnet. Dazu kann der Hutteil reib- und/oder formschlüssig an dem ersten Kupplungselement oder dem zweiten Kupplungselement angeordnet sein. Insbesondere wenn der Hutteil abnehmbar ausgebildet ist, kann der Sicherungsring einstückig mit dem ersten Kupplungselement oder dem zweiten Kupplungselement ausgebildet sein. Der Sicherungsring kann eine oder mehrere, vorzugsweise parallel zur Rotationsachse angeordnete, Durchgangsbohrungen aufweisen.

Die Kupplungseinheit ist vorzugsweise in das Einleitelement und/oder das Ausleitelement einrastbar und/oder einschraubbar ausgebildet. Insbesondere um die Kupplungseinheit einrastbar auszubilden, kann die Kupplungseinheit an ihrer Außenkontur eine, vorzugsweise um die Rotationsachse verlaufende, Ringnut zum Eingriff eines Rastvorsprungs aufweisen. Der Rastvorsprung kann ein Dichtelement umfassen, das seinerseits in einer umlaufenden Nut an dem Einleitelement und/oder dem Ausleitelement angeordnet ist. Der Rastvorsprung kann somit eine zusätzliche Dichtfunktion aufweisen.

Eine erfindungsgemäße Griffeinheit, insbesondere für ein chirurgisches Instrument, umfasst ein distales Ende, ein proximales Ende, eine das distale Ende und das proximale Ende verbindende Grifflängsachse und einen zuvor beschriebenen Drehmomentbegrenzer, wobei das Einleitelement eine Greiffläche aufweist und die Kupplungseinheit in und/oder an dem proximalen Ende angeordnet ist. Als proximales Ende wird hier und im Folgenden vorzugsweise das Ende der Griffeinheit bezeichnet, das bei bestimmungsgemäßer Benutzung der Griffeinheit dem Anwender zugewandt angeordnet ist. Als distales Ende wird hier und im Folgenden dementsprechend vorzugsweise das Ende der Griffeinheit bezeichnet, das bei bestimmungsgemäßer Benutzung der Griffeinheit dem Anwender abgewandt angeordnet ist. Dadurch kann insbesondere einem unbeabsichtigten Entfernen der Kupplungseinheit vorgebeugt und der Verschmutzungseintrag in die Kupplungseinheit sowie die gesamte Griffeinheit reduziert werden. Die Greiffläche ist vorzugsweise ergonomisch geformt, besonders bevorzugt für eine menschliche Hand, und kann zumindest abschnittsweise aus Kunststoff, insbesondere aus Silikon, ausgebildet sein. Insbesondere wenn die Griffeinheit für ein chirurgisches Instrument vorgesehen und geeignet ist, umfasst das Ausleitelement vorzugsweise eine Antriebswelle für das chirurgische Instrument.

In einer bevorzugten Ausführungsform der Griffeinheit ist die Kupplungseinheit derart in der Griffeinheit anordenbar, dass die Rotationsachse entlang der Grifflängsachse angeordnet ist. Besonders bevorzugt ist die Rotationsachse auf der Grifflängsachse angeordnet.

Ein erfindungsgemäßes chirurgisches Instrument umfasst einen zuvor beschriebenen Drehmomentbegrenzer oder eine zuvor beschriebene Griffeinheit.

Ein Ausführungsbeispiel der Erfindung wird anhand der nachfolgenden Figuren erläutert. Es zeigt:
- Figur 1: eine Seitenansicht eines ersten Ausführungsbeispiels einer Griffeinheit mit einem Drehmomentbegrenzer,
- Figur 2: eine Explosionsdarstellung des in Fig. 1 gezeigten Ausführungsbeispiels,
- Figur 3: einen Längsschnitt des in Fig. 1 gezeigten Ausführungsbeispiels,
- Figur 4: eine perspektivische Detailansicht des in Fig. 3 gezeigten Längsschnitts,
- Figur 5: eine perspektivische Darstellung der Kupplungseinheit des in Fig. 1 gezeigten Ausführungsbeispiels,
- Figur 6: eine Explosionsdarstellung der in Fig. 5 gezeigten Kupplungseinheit,
- Figur 7: eine perspektivische Darstellung eines Längsschnitts der in Fig. 5 gezeigten Kupplungseinheit,
- Figur 8: eine perspektivische Darstellung eines Querschnitts der in Fig. 5 gezeigten Kupplungseinheit,
- Figur 9: eine Explosionsansicht eines zweiten Ausführungsbeispiels einer Griffeinheit mit einem Drehmomentbegrenzer,
- Figur 10: eine perspektivische Schnittansicht des in Fig. 9 gezeigten Ausführungsbeispiels,
- Figur 11: eine Explosionsdarstellung eines alternativen Ausführungsbeispiels einer Kupplungseinheit,
- Figur 12: eine perspektivische Darstellung eines Längsschnitts der in Fig. 11 gezeigten Kupplungseinheit,
- Figur 13: eine Darstellung eines Querschnitts der in Fig. 11 gezeigten Kupplungseinheit.

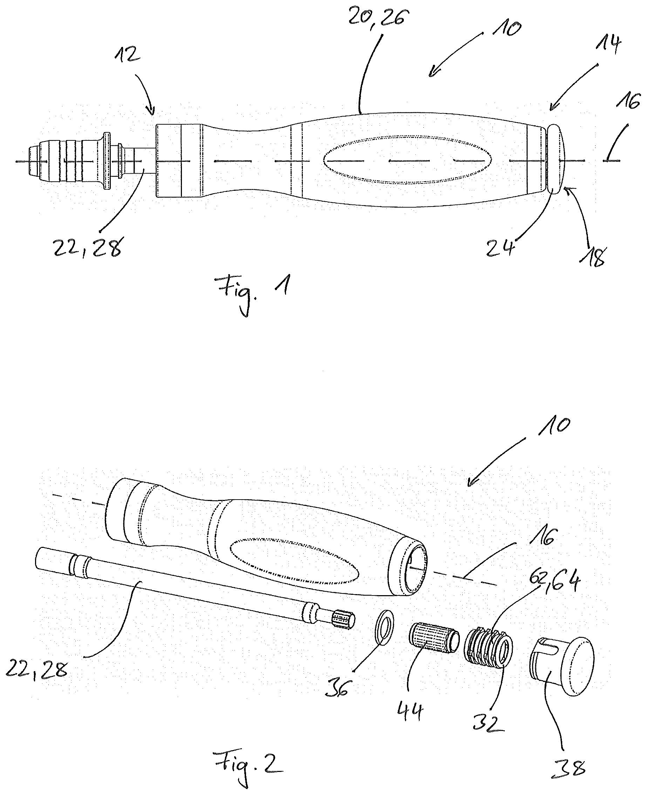

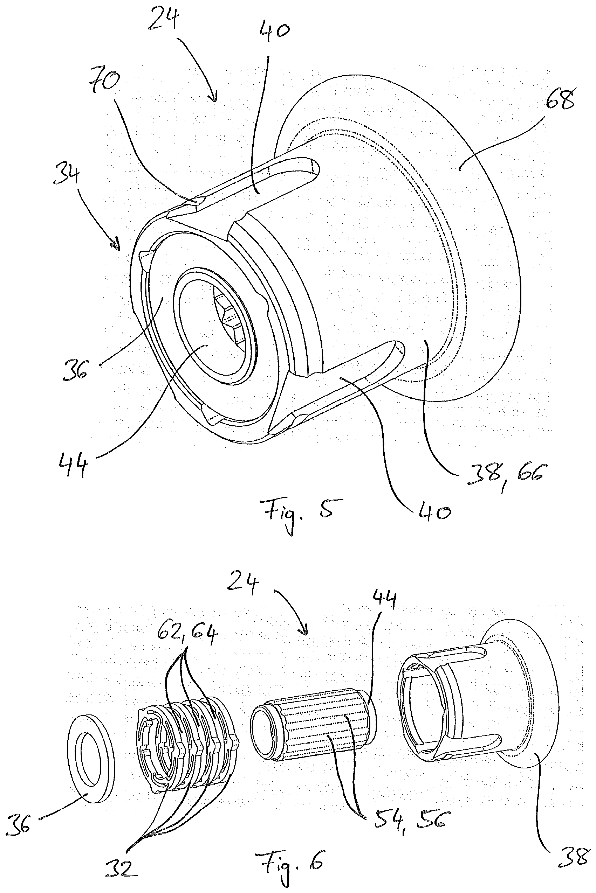

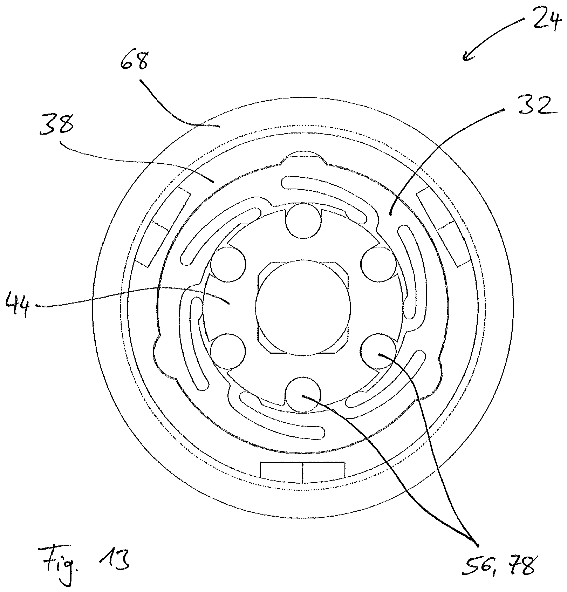

Die Figuren 1 bis 13 zeigen verschiedene Ansichten verschiedener Ausführungsbeispiele. Der Übersichtlichkeit halber werden nicht alle Bezugszeichen in jeder Figur verwendet. Für gleiche Teile werden die gleichen Bezugszeichen verwendet.

In den Fig. 1-4 ist ein erstes Ausführungsbeispiel einer erfindungsgemäßen Griffeinheit 10 dargestellt, die insbesondere für ein chirurgisches Instrument vorgesehen und geeignet ist.

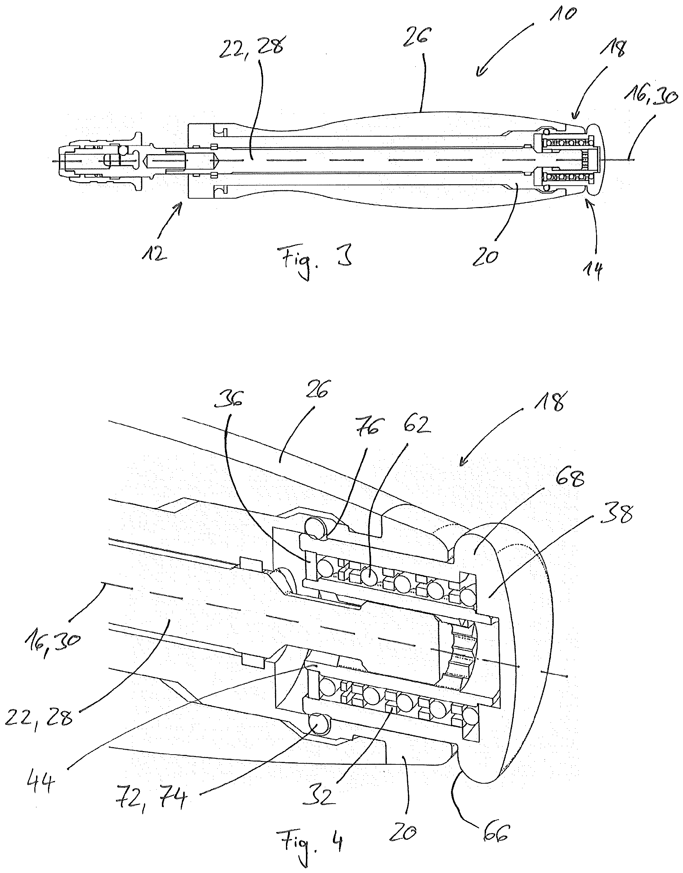

Die Griffeinheit 10 weist ein distales Ende 12, ein proximales Ende 14 sowie eine das distale Ende 12 und das proximale Ende 14 verbindende Grifflängsachse 16 auf. Die Griffeinheit 10 umfasst ferner einen Drehmomentbegrenzer 18 mit einem Einleitelement 20 zum Einleiten von Drehmoment in den Drehmomentbegrenzer 18, einem Ausleitelement 22 zum Ausleiten von Drehmoment aus dem Drehmomentbegrenzer 18, und einer Kupplungseinheit 24, mittels der das Einleitelement 20 mit dem Ausleitelement 22 koppelbar ist. Die Kupplungseinheit 24 und deren Anordnung in der Griffeinheit 10 sind insbesondere in den Fig. 4-8 detaillierter dargestellt.

In den Darstellungen in Fig. 1-4 ist besonders gut zu erkennen, dass das Einleitelement 20 eine Greiffläche 26 aufweist, und die Kupplungseinheit 24 in und an dem proximalen Ende 14 der Griffeinheit 10 angeordnet ist. Die Greiffläche 26 ist vorzugsweise ergonomisch geformt und kann zumindest abschnittsweise aus Kunststoff, insbesondere aus Silikon, ausgebildet sein. Insbesondere wenn die Griffeinheit 10 für ein chirurgisches Instrument vorgesehen und geeignet ist, umfasst das Ausleitelement 22 vorzugsweise eine Antriebswelle 28 für das chirurgische Instrument. In einer bevorzugten Ausführungsform der Griffeinheit 10 ist die Kupplungseinheit 24 derart in der Griffeinheit 10 anordenbar, dass eine Rotationsachse 30 des Drehmomentbegrenzers 18 entlang der Grifflängsachse 16 angeordnet ist. Besonders bevorzugt ist die Rotationsachse 30 auf der Grifflängsachse 16 angeordnet.

Die Kupplungseinheit 24 ist vorzugsweise auswechselbar in dem Drehmomentbegrenzer 18 angeordnet. Bevorzugt ist die Kupplungseinheit 24 ohne spezielle Montagevorrichtung und/oder mit Standardwerkzeug auswechselbar. Die Kupplungseinheit 24 kann insbesondere werkzeuglos auswechselbar sein. Besonders bevorzugt ist die Kupplungseinheit 24 anwenderseitig auswechselbar in dem Drehmomentbegrenzer 18 angeordnet. Vorzugsweise ist die Kupplungseinheit 24 vollständig, also mit allen zu der Kupplungseinheit 24 zählenden Komponenten, auswechselbar ausgebildet.

Vorzugsweise ist der Drehmomentbegrenzer 18 derart ausgebildet, dass das ein auf das Einleitelement 20 aufgebrachtes Drehmoment unterhalb der Drehmomentgrenze vom Einleitelement 20 auf das Ausleitelement 22 übertragbar ist. Ein die Drehmomentgrenze übersteigender Betrag des am Einleitelement 20 anliegenden Drehmoments ist vorzugsweise hingegen nicht von dem Einleitelement 20 auf das Ausleitelement 22 übertragbar.

Wie unter anderem Fig. 2 u. 6 zeigen, weist die Kupplungseinheit 24 mindestens einen Kupplungskörper 32 auf, der zur kraft- und formschlüssigen Drehmomentübertragung ausgebildet sein kann. Der mindestens eine Kupplungskörper 32 ist vorzugsweise durch mehrere Kupplungskörper 32, im vorliegenden Ausführungsbeispiel durch vier Kupplungskörper 32, gebildet. Die Kupplungskörper 32 können entlang der Rotationsachse 30 nacheinander angeordnet sein. Durch die Anzahl der verwendeten Kupplungskörper 32 kann, insbesondere werkseitig, die Drehmomentgrenze einstellbar sein. An mindestens einem axialen Ende 34 der Kupplungseinheit 24 kann ein Sicherungsring 36 zur axialen Sicherung der Kupplungskörper 32 angeordnet sein.

Die Kupplungseinheit 24 kann eine, um die Rotationsachse 30 angeordnete, kreisringförmige Grundform aufweisen. Vorzugsweise weist damit auch jeder der Kupplungskörper 32 eine kreisringförmige Grundform auf. Bevorzugt ist das Einleitelement 20 dabei bezüglich der Rotationsachse 30 radial außerhalb der Kupplungseinheit 24, das Ausleitelement 22 bezüglich der Rotationsachse 30 radial innerhalb der Kupplungseinheit 24 angeordnet (Fig. 4). Insbesondere um den konstruktiven Aufbau des Drehmomentbegrenzers 18 zu vereinfachen, kann die Kupplungseinheit 24 entlang der Rotationsachse 30 in dem Drehmomentbegrenzer 18 angeordnet sein.

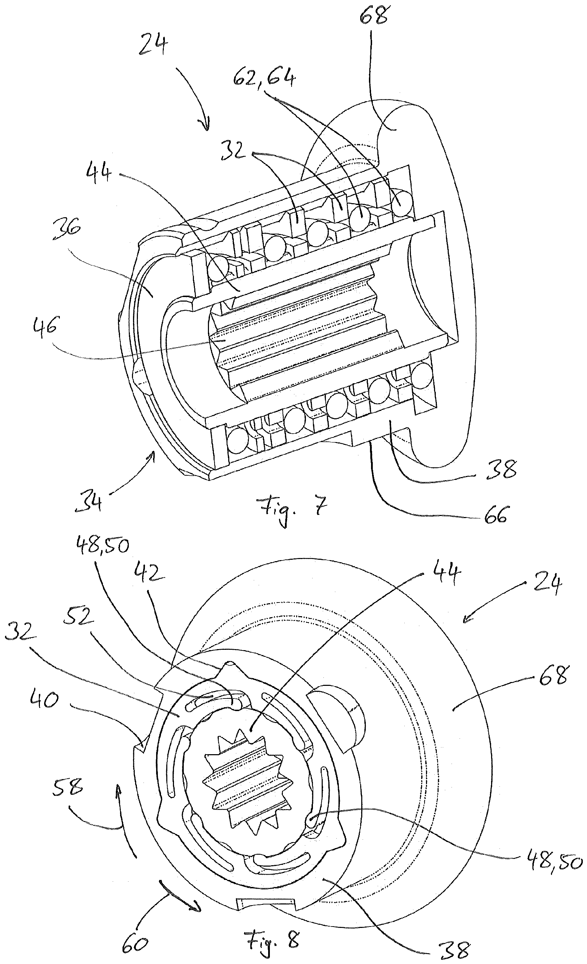

In den Fig. 4-8 ist die Kupplungseinheit 24 detaillierter dargestellt. Die Kupplungseinheit 24 weist vorzugsweise ein erstes Kupplungselement 38 auf, das, wie Fig. 8 zeigt, einerseits mit den Kupplungskörpern 32 sowie andererseits mittels Längsnuten 40 mit dem Einleitelement 20 drehfest und unmittelbar koppelbar ist. Fig. 4 zeigt dabei, dass das erste Kupplungselement 38 vorzugsweise durch ein von dem Einleitelement 20 verschiedenes Bauteil gebildet ist und unmittelbar mit dem Einleitelement 20 gekoppelt ist. Eine bevorzugte Ausführungsform der drehfesten Kopplung ist eine formschlüssige Verbindung mittels einer ersten Keilverzahnung 42.

Insbesondere wenn, wie es im gezeigten Ausführungsbeispiel vorzugsweise der Fall ist, das erste Kupplungselement 38 radial außerhalb der Kupplungskörper 32 angeordnet ist, kann das erste Kupplungselement 38 für die Kupplungseinheit 24 eine Gehäusefunktion aufweisen. Dadurch kann die Auswechselbarkeit der Kupplungseinheit 24 verbessert werden.

Die Kupplungseinheit 24 kann ferner ein zweites Kupplungselement 44 aufweisen, das ebenfalls unter anderem in Fig. 8 dargestellt ist. Das zweite Kupplungselement 44 kann einerseits mit den Kupplungskörpern 32 drehmomentabhängig und unmittelbar sowie andererseits mit dem Ausleitelement 22 drehfest und unmittelbar koppelbar sein. Die drehfeste Kopplung des zweiten Kupplungselements 44 mit dem Ausleitelement 22 ist vorzugsweise ebenfalls durch eine formschlüssige Verbindung, nämlich mittels einer zweiten Keilverzahnung 46, realisiert (siehe insbesondere Fig. 4).

Durch die drehmomentabhängige Kopplung kann mittels der Wirkverbindung der Kupplungskörper 32 mit dem zweiten Kupplungselement 44 die Drehmomentgrenze des Drehmomentbegrenzers 18 definiert sein. Die drehmomentabhängige Kopplung kann dabei mittels einer unmittelbaren form- und kraftschlüssigen Verbindung realisiert sein, die vorzugsweise bei Überschreiten der Drehmomentgrenze nach dem Prinzip einer Rutschkupplung aufgehoben wird. Durch das Vorsehen des zweiten Kupplungselements 44 kann insbesondere der dabei auftretende Verschleiß an dem zweiten Kupplungselement 44 stattfinden, sodass die Lebensdauer des Ausleitelements 22 dadurch nicht beeinträchtigt wird.

Zur Realisierung der drehmomentabhängigen Kopplung der Kupplungskörper 32 mit dem zweiten Kupplungselement 44 weist vorzugsweise jeder der Kupplungskörper 32 mindestens ein als Federarm 48 ausgebildetes Andrückelement 50 auf. Besonders bevorzugt weist jeder der Kupplungskörper 32 eine Vielzahl von Federarmen 48, beispielsweise sechs Federarme 48, auf, die beispielsweise in Fig. 8 gut zu erkennen sind. Bevorzugt liegt jeder der Federarme 48 an dem zweiten Kupplungselement 44 an und stellt so die Wirkverbindung zwischen dem jeweiligen Kupplungskörper 32 und dem zweiten Kupplungselement 44 her. Jeder der Federarme 48 kann sich durch einen Spalt 52, der insbesondere L-förmig ausgebildet sein kann, von dem übrigen Kupplungskörper 32 abheben. Mittels des Spalts 52 ist vorzugsweise die federnde Charakteristik des jeweiligen Federarms 48 realisiert.

Das zweite Kupplungselement 44 kann mindestens ein, vorzugsweise als Vertiefung 54 ausgebildetes, Rastelement 56 zum Zusammenwirken mit einem der Federarme 48 aufweisen. Die Kontur jeder der Federarme 48 entspricht vorzugsweise zumindest abschnittsweise der Kontur der mindestens einen Vertiefung 54. Das zweite Kupplungselement 44 kann eine Vielzahl an Vertiefungen 54 aufweisen. Die Anzahl der Vertiefungen 54 des zweiten Kupplungselements 44 kann ein ganzzahlig Vielfaches, beispielsweise das Doppelte, der Anzahl an Federarmen 48 einer der Kupplungskörper 32 sein. So kann jeder der Federarme 48 mit einer der Vertiefungen 54 in unmittelbare Wirkverbindung gebracht werden. Eine permanente Wirkverbindung zwischen den Federarmen 48 und den Vertiefungen 54 besteht vorzugsweise in einem eingerasteten Zustand, also unterhalb der Drehmomentgrenze.

Wie insbesondere in Fig. 8 gezeigt ist, können die Federarme 48 sämtlich in einer ersten Umfangsrichtung 58 um die Rotationsachse 30 ausgerichtet sein. So kann eine erste Drehmomentgrenze in der ersten Umfangsrichtung 58 um die Rotationsachse 30 gegenüber einer zweiten Drehmomentgrenze in einer zweiten Umfangrichtung 60 um die Rotationsachse 30 unterschiedlich hoch ausgebildet sein. Wird der Drehmomentbegrenzer 18 beispielsweise in einem Schraubendreher eingesetzt, kann so zum Beispiel ein maximales Anzugsmoment kleiner festgelegt werden als ein maximales Lösemoment. Im gezeigten Ausführungsbeispiel liegt die Drehmomentgrenze bei einer Drehung der Kupplungskörper 32 gegenüber dem zweiten Kupplungselement 44 in der ersten Umfangsrichtung 58 höher als in der zweiten Umfangsrichtung 60.

Wie insbesondere die Fig. 4 u. 7 zeigen, umfasst die Kupplungseinheit 24 vorzugsweise mehrere, jeweils als Gummiring 62 ausgebildete Distanzelemente 64, die entlang der Rotationsachse 30 im Wechsel mit den Kupplungskörpern 32 angeordnet sind. Die Gummiringe 62 können zur Dämpfung, insbesondere der Federarme 48, sowie zur Geräuschminderung beitragen, sowie eine, zumindest leichte, Erhöhung der Drehmomentgrenze bewirken. Darüber hinaus kann jeder der Gummiringe 62 eine axiale Montagesicherung für den jeweils zuvor montierten Kupplungskörper 32 darstellen.

Vor allem die Fig. 4-8 zeigen, dass die Kupplungseinheit 24 derart ausgebildet sein kann, dass sie eine im Wesentlichen pilzförmige Außenkontur 66 aufweist. Im montierten Zustand der Kupplungseinheit 24 ist die Pilzform vorzugsweise um die Rotationsachse 30 des Drehmomentbegrenzers 18 angeordnet. Ein Hutteil 68 der pilzförmigen Außenkontur 66 kann besonders gut zu greifen sein, was die Auswechselbarkeit der Kupplungseinheit 24 erleichtern kann. Die pilzförmige Außenkontur 66 ist vorzugsweise dadurch gebildet, dass das erste Kupplungselement 38, pilzförmig ausgebildet ist.

Die Kupplungseinheit 24 kann in das Einleitelement 20 und/oder das Ausleitelement 22 einrastbar und/oder einschraubbar ausgebildet sein. Insbesondere um die Kupplungseinheit 24 einrastbar auszubilden, kann die Kupplungseinheit 24 an ihrer Außenkontur 66 eine, vorzugsweise um die Rotationsachse 30 verlaufende, Ringnut 70 zum Eingriff eines Rastvorsprungs 72 aufweisen. Der Rastvorsprung 72 kann ein Dichtelement 74 umfassen, das seinerseits in einer umlaufenden Nut 76 an dem Einleitelement 20 angeordnet ist. Der Rastvorsprung 72 kann somit eine zusätzliche Dichtfunktion aufweisen.

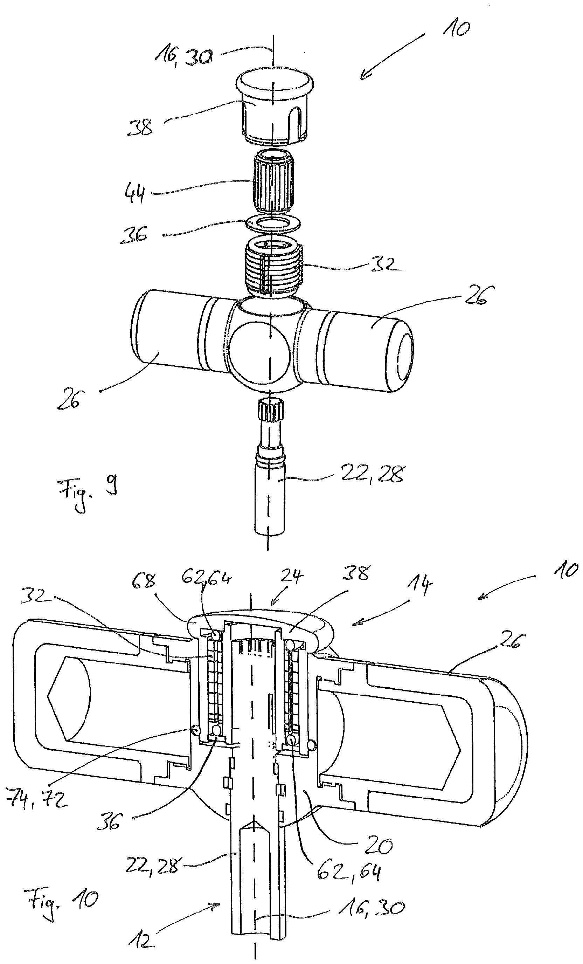

Die Fig. 9-10 zeigen ein zweites Ausführungsbeispiel einer Griffeinheit, die in weiten Teilen entsprechend dem in den Fig. 1-8 gezeigten Ausführungsbeispiel aufgebaut ist. Im Folgenden soll daher im Wesentlichen auf die Unterschiede gegenüber dem ersten Ausführungsbeispiel eingegangen werden.

Wie aus den Fig. 9 u. 10 erkennbar ist, kann Griffeinheit 10 T-förmig ausgebildet sein, insbesondere indem die Greiffläche 26 senkrecht zu der Grifflängsachse 16 angeordnet ist. Die Formgebung des Einleitelements 20 kann entsprechend angepasst sein. Derartige Ausführungsformen der Griffeinheit 10 können beispielsweise für Anwendungsfälle eingesetzt werden, bei denen ein hohes Drehmoment in die Griffeinheit 10 eingeleitet werden soll.

Insbesondere Fig. 10 zeigt, dass die in dem Drehmomentbegrenzer 18 angeordneten Kupplungskörper 32 entlang der Grifflängsachse 16 unmittelbar aneinander angrenzend angeordnet sein können. Die Distanzelemente 62, 64 können derart angeordnet sein, dass sie lediglich das aus den Kupplungskörpern 32 gebildete Paket entlang der Grifflängsachse 16 in beiden axialen Richtungen abschließen. Trotz dieses Unterscheidungsmerkmals gegenüber dem ersten Ausführungsbeispiel, Unterscheidet sich der grundlegende konstruktive Aufbau der Kupplungseinheit 24 und/oder die Schnittstellen, insbesondere das erste Kupplungselement 38 und das zweite Kupplungselement 44, im ersten Ausführungsbeispiel und um zweiten Ausführungsbeispiel vorzugsweise nicht. Damit werden weitere Möglichkeiten der auswechselbaren Kupplungseinheit 24 deutlich: Die gleiche Kupplungseinheit 24 kann in unterschiedlichen Griffeinheiten 10 anordenbar sein und dieselbe Griffeinheit 10 kann mit unterschiedlichen Kupplungseinheiten 24 bestückbar sein.

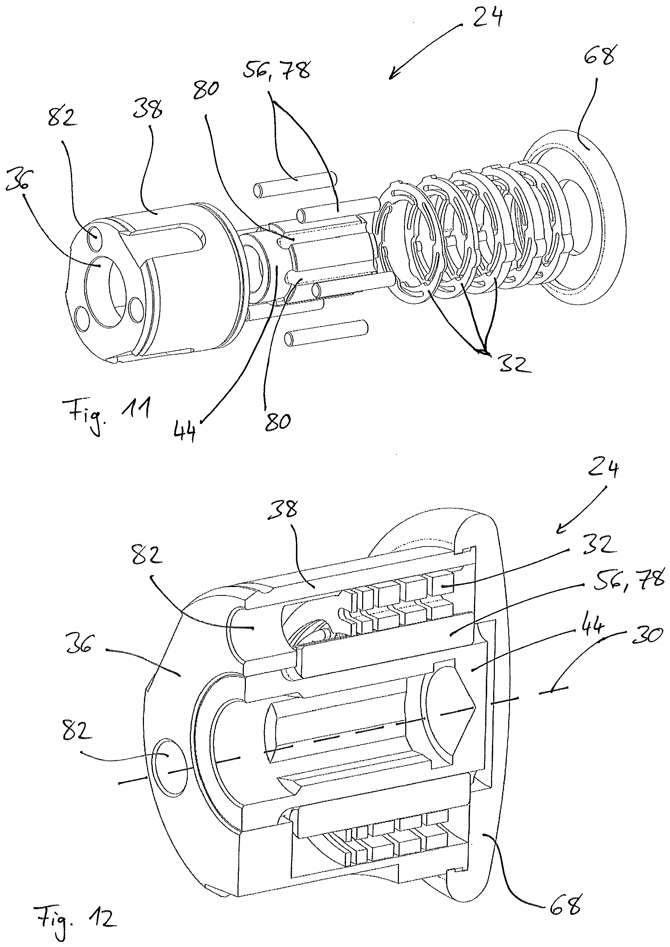

Die Fig. 11-13 zeigen ein alternatives Ausführungsbeispiel der Kupplungseinheit 24. Von der in den Fig. 1-10 gezeigten Kupplungseinheit 24 unterscheidet sich die Kupplungseinheit 24 der Fig. 11-13 unter anderem darin, dass die Rastelemente 56 vorzugsweise als zylindrische Walzenkörper 78 ausgebildet sind. Die Walzenkörper 78 sind vorzugsweise parallel zu der Rotationsachse 30 des Drehmomentbegrenzers 18 und gleichmäßig verteilt um die Rotationsachse 30 angeordnet. Vorzugsweise weist das zweite Kupplungselement eine der Anzahl von Walzenkörpern 78 entsprechende Anzahl von Ausnehmungen 80 auf, wobei in jeder der Ausnehmungen 80 jeweils einer der Walzenkörper 78 angeordnet sein kann.

Wie insbesondere Fig. 12 zeigt, kann der Hutteil 68 axial abnehmbar an dem ersten Kupplungselement 38 angeordnet sein. Dazu kann der Hutteil 68 reib- und/oder formschlüssig an dem ersten Kupplungselement 38 angeordnet sein. Ferner kann der Sicherungsring 36 einstückig mit dem ersten Kupplungselement 38 ausgebildet sein. Das erste Kupplungselement 38 kann somit topfförmig ausgebildet sein. Der Sicherungsring 38 kann eine oder mehrere, vorzugsweise parallel zur Rotationsachse 30 angeordnete, Durchgangsbohrungen 82 aufweisen.

### Bezugszeichenliste

- 10: Griffeinheit
- 12: distales Ende
- 14: proximales Ende
- 16: Grifflängsachse
- 18: Drehmomentbegrenzer
- 20: Einleitelement
- 22: Ausleitelement
- 24: Kupplungseinheit
- 26: Greiffläche
- 28: Antriebswelle
- 30: Rotationsachse
- 32: Kupplungskörper
- 34: axiales Ende
- 36: Sicherungsring
- 38: erstes Kupplungselement
- 40: Längsnut
- 42: erste Keilverzahnung
- 44: zweites Kupplungselement
- 46: zweite Keilverzahnung
- 48: Federarm
- 50: Andrückelement
- 52: Spalt
- 54: Vertiefung
- 56: Rastelement
- 58: erste Umfangsrichtung
- 60: zweite Umfangsrichtung
- 62: Gummiring
- 64: Distanzelement
- 66: Außenkontur
- 68: Hutteil
- 70: Ringnut
- 72: Rastvorsprung
- 74: Dichtelement
- 76: umlaufende Nut
- 78: Walzenkörper
- 80: Ausnehmung
- 82: Durchgangsbohrung

## Patentansprüche

1. Drehmomentbegrenzer (18), insbesondere für ein chirurgisches Instrument, mit einer Rotationsachse (30), einem Einleitelement (20) zum Einleiten von Drehmoment in den Drehmomentbegrenzer (18), einem Ausleitelement (22) zum Ausleiten von Drehmoment aus dem Drehmomentbegrenzer (18), und einer mindestens einen Kupplungskörper (32) aufweisenden Kupplungseinheit (24), mittels der das Einleitelement (20) mit dem Ausleitelement (22) koppelbar ist,
**dadurch gekennzeichnet, dass** die Kupplungseinheit (24) auswechselbar in dem Drehmomentbegrenzer (18) angeordnet ist.

2. Drehmomentbegrenzer nach Anspruch 1,
**dadurch gekennzeichnet, dass** die Kupplungseinheit (24) entlang der Rotationsachse (30) in und/oder an dem Drehmomentbegrenzer (18) angeordnet ist.

3. Drehmomentbegrenzer nach einem der vorhergehenden Ansprüche,
**dadurch gekennzeichnet, dass** die Kupplungseinheit (24) ein erstes Kupplungselement (38) aufweist, das einerseits mit dem mindestens einen Kupplungskörper (32) sowie andererseits mit dem Einleitelement (20) oder dem Ausleitelement (22) drehfest und/oder unmittelbar koppelbar ist.

4. Drehmomentbegrenzer nach einem der vorhergehenden Ansprüche,
**dadurch gekennzeichnet, dass** die Kupplungseinheit (24) ein zweites Kupplungselement (44) aufweist, das einerseits mit dem mindestens einen Kupplungskörper (32) drehmomentabhängig und/oder unmittelbar sowie andererseits mit dem Einleitelement (20) oder dem Ausleitelement (22) drehfest und/oder unmittelbar koppelbar ist.

5. Drehmomentbegrenzer nach einem der vorhergehenden Ansprüche,
**dadurch gekennzeichnet, dass** der mindestens eine Kupplungskörper (32) mindestens ein, vorzugsweise als Federarm (48) ausgebildetes, Andrückelement (50) aufweist.

6. Drehmomentbegrenzer nach den Ansprüchen 4 und 5,
**dadurch gekennzeichnet, dass** das zweite Kupplungselement (44) mindestens ein Rastelement (56) zum Zusammenwirken mit dem mindestens einen Andrückelement (50) aufweist.

7. Drehmomentbegrenzer nach Anspruch 6,
**dadurch gekennzeichnet, dass** das mindestens eine Rastelement als Walzenkörper ausgebildet ist, der in einer Ausnehmung des zweiten Kupplungselements angeordnet ist.

8. Drehmomentbegrenzer nach einem der Ansprüchen 5 bis 7,
**dadurch gekennzeichnet, dass** das mindestens eine Andrückelement (50) durch eine Vielzahl von Andrückelementen (50) gebildet ist, die sämtlich in einer selben Umfangsrichtung (58, 60) um die Rotationsachse (30) ausgerichtet sind.

9. Drehmomentbegrenzer nach einem der vorhergehenden Ansprüche,
**dadurch gekennzeichnet, dass** der mindestens eine Kupplungskörper (32) durch mehrere Kupplungskörper (32) gebildet ist, die entlang der Rotationsachse (30) nacheinander angeordnet sind.

10. Drehmomentbegrenzer nach einem der vorhergehenden Ansprüche,
**dadurch gekennzeichnet, dass** die Kupplungseinheit (24) mindestens ein, vorzugsweise als Gummiring (62) ausgebildetes, Distanzelement (64) umfasst, das entlang der Rotationsachse (30) angrenzend an einen des mindestens einen Kupplungskörpers (32) angeordnet ist.

11. Drehmomentbegrenzer nach einem der vorhergehenden Ansprüche,
**dadurch gekennzeichnet, dass** die Kupplungseinheit (24) eine im Wesentlichen pilzförmige Außenkontur (66) aufweist.

12. Drehmomentbegrenzer nach einem der vorhergehenden Ansprüche,
**dadurch gekennzeichnet, dass** die Kupplungseinheit (24) in das Einleitelement (20) und/oder das Ausleitelement (22) einrastbar und/oder einschraubbar ausgebildet ist.

13. Griffeinheit (10), insbesondere für ein chirurgisches Instrument, umfassend ein distales Ende (12), ein proximales Ende (14), eine das distale Ende (12) und das proximale Ende (14) verbindende Grifflängsachse (16) und einen Drehmomentbegrenzer (18) nach einem der vorhergehenden Ansprüche, wobei das Einleitelement (20) eine Greiffläche (26) aufweist und die Kupplungseinheit (24) in und/oder an dem proximalen Ende (14) angeordnet ist.

14. Griffeinheit nach Anspruch 13 mit einem Drehmomentbegrenzer nach einem der Ansprüche 1 bis 12,
**dadurch gekennzeichnet, dass** die Kupplungseinheit (24) derart in der Griffeinheit (10) anordenbar ist, dass die Rotationsachse (30) entlang der Grifflängsachse (16) angeordnet ist.

15. Chirurgisches Instrument mit einem Drehmomentbegrenzer (18) nach einem der Ansprüche 1 bis 12 oder einer Griffeinheit (10) nach einem der Ansprüche 13 bis 14.
